(19) 

**Europäisches Patentamt**
**European Patent Office**
**Office européen des brevets**

(11) **EP 4 378 489 A1**

(12) **EUROPEAN PATENT APPLICATION**
published in accordance with Art. 153(4) EPC

(43) Date of publication:
**05.06.2024  Bulletin 2024/23**

(21) Application number: **22922283.1**

(22) Date of filing: **22.03.2022**

(51) International Patent Classification (IPC):
*A61L 17/10* (2006.01)  *A61L 17/12* (2006.01)
*A61L 17/00* (2006.01)  *A61B 17/06* (2006.01)
*A61B 17/00* (2006.01)

(52) Cooperative Patent Classification (CPC):
**A61L 17/10; A61B 17/00; A61B 17/06;**
**A61L 17/00; A61L 17/12; C08L 5/08**

(86) International application number:
**PCT/KR2022/003958**

(87) International publication number:
**WO 2023/140422 (27.07.2023 Gazette 2023/30)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **21.01.2022  KR 20220009055**

(71) Applicant: **Curepharmtech Co., Ltd.**
**Goyang-si, Gyeonggi-do 10401 (KR)**

(72) Inventor: **KIM, Dae Young**
**Goyang-si Gyeonggi-do 10307 (KR)**

(74) Representative: **M. Zardi & Co S.A.**
**Via G. B. Pioda, 6**
**6900 Lugano (CH)**

(54) **ABSORBABLE SUTURE CONTAINING HYALURONIC ACID AND MANUFACTURING METHOD THEREFOR**

(57)  The present disclosure relates to an absorbable suture containing hyaluronic acid and a method for manufacturing the same. The absorbable suture includes an absorbent polymer including at least one material selected from the group consisting of poly[L-lactic acid] (PLLA), poly[lactic acid] (PLA), poly[p-dioxanone] (PDO), poly[ε-caprolactone] (PCL), poly[glycolic acid] (PGA), poly[glycolic acid- co-ε-caprolactone (PGCL), poly[L-lactic-co-glycolic acid (PLGA), poly[L-lactide-co-ε-caprolactone] (PLCL) copolymer, poly[p-dioxanone-co-ε-caprolactone] (PDCL) copolymer, and poly[L-lactic acid-co-p-dioxanone] (PLDA) copolymer, and hyaluronic acid contained in the absorbent polymer, wherein the hyaluronic acid is included in the absorbable suture in an amount range of more than 0.1% by weight to less than 5.0% by weight based on the total weight.

EP 4 378 489 A1

**Description**

**BACKGROUND**

Field

The present disclosure relates to an absorbable suture containing hyaluronic acid in an absorbent polymer and a method for manufacturing the same.

Related Art

In general, a suture is used as a thread for suturing a human body region damaged by surgery or the like, and a human body region is sutured with the suture by connecting an injection needle to an end of the suture and penetrating a part of the human body through this. Sutures are divided into non-absorbable sutures, in which a procedure for removing this is proceeded after damaged regions are completely sutured as a certain period of time has elapsed after surgery, and absorbable sutures that are biodegraded and disappear on their own in the human body after a certain period of time has elapsed, wherein the absorbable sutures are generally manufactured using a material expressed as a biodegradable resin, an absorbent resin, or the like. Absorbable sutures do not require acts such as a separate procedure for removal, and are spontaneously decomposed and disappear so that they are widely used recently due to their convenience, and there is a tendency of increasing their market share worldwide, and currently, the world market share thereof is already about 80 to 90%.

Sutures are divided into monofilaments of a single structure and a multifilament of a complex structure. Although flexibility is secured in the case of the multifilament, due to the nature of the structure, it is easy for bacteria to propagate so that there is a disadvantage in that it is relatively easy to be contaminated, whereas sutures composed of monofilaments have the advantage of not being relatively easily contaminated. Meanwhile, sutures should maintain certain levels or more of knot strength and elongation to firmly seal and maintain damaged regions of the human body, but since absorbable sutures using absorbent polymers may have lower strength than non-absorbable sutures due to their characteristics of being naturally decomposed after a certain period of time has elapsed, the need for knot strength or elongation to be maintained at a certain numerical level or more is particularly being further emphasized.

Meanwhile, general sutures only function to suture simple damaged wounds and maintain sutures, and it has been common for damaged human body wounds to be sutured by tissue regeneration on their own. Accordingly, there is a need for a study on a suture that allows the suture itself to perform additional various functions in addition to the suture function.

**SUMMARY**

Accordingly, an object to be solved by the present disclosure is to provide an absorbable suture capable of imparting a moisturizing effect to the skin in addition to being decomposed in the body, and a method for manufacturing the same.

Another object of the present disclosure is to provide an absorbable suture capable of releasing hyaluronic acid continuously and in an appropriate amount even after a long time has elapsed after performing a suture procedure by including hyaluronic acid not only on the surface of the absorbable suture but also inside the absorbable suture, and a method for manufacturing the same.

Another object of the present disclosure is to provide an absorbable suture capable of preventing deterioration in knot strength, elongation, etc., which are physical properties required of an absorbable suture while including hyaluronic acid in the absorbable suture, and a method for manufacturing the same.

The problems of the present disclosure are not limited to the above-described contents. The problems of the present disclosure will be understood from the entire contents of this specification, and those of ordinary skill in the art to which the present disclosure belongs will have no difficulty in understanding the additional problems of the present disclosure.

An absorbable suture according to one embodiment of the present disclosure for solving the above-described problems includes an absorbent polymer including at least one material selected from the group consisting of poly[L-lactic acid] (PLLA), poly[lactic acid] (PLA), poly[p-dioxanone] (PDO), poly[ε-caprolactone] (PCL), poly[glycolic acid] (PGA), poly[glycolic acid- co-ε-caprolactone (PGCL), poly[L-lactic-co-glycolic acid (PLGA), poly[L-lactide-co-ε-caprolactone] (PLCL) copolymer, poly[p-dioxanone-co-ε-caprolactone] (PDCL) copolymer, and poly[L-lactic acid-co-p-dioxanone] (PLDA) copolymer, and hyaluronic acid contained in the absorbent polymer, wherein the hyaluronic acid is included in the absorbable suture in an amount range of more than 0.1% by weight to less than 5.0% by weight based on the total weight.

Further, the absorbable suture may be composed of a monofilament, and the hyaluronic acid may be distributed inside and on the surface of the monofilament.

Further, the hyaluronic acid may be included in a range of 0.5% by weight or more to less than 5.0% by weight of the total weight of the absorbable suture.

Further, the hyaluronic acid may be included in a range of 0.5% by weight or more to 4.0% by weight or less of the total weight of the absorbable suture.

Further, the hyaluronic acid may be included in a range of 0.5% by weight or more to 3.0% by weight or less of the total weight of the absorbable suture.

Further, the absorbable suture may have a breaking strength retention (BSR) range of 40% or more to 80% or less after 2 weeks after transplantation.

Further, the absorbent polymer is composed of poly[p-dioxanone] (PDO), and poly[p-dioxanone] (PDO) may have a melting index (MI) range of 2 g/10 min or more to 16 g/10 min or less.

Meanwhile, a method for manufacturing an absorbable suture according to one embodiment of the present disclosure for solving the above-described problems includes the steps of preparing an absorbent polymer raw material including at least one material selected from the group consisting of poly[L-lactic acid] (PLLA), poly [lactic acid] (PLA), poly[p-dioxanone] (PDO), poly[$\epsilon$-caprolactone] (PCL), poly[glycolic acid] (PGA), poly[glycolic acid- co-$\epsilon$-caprolactone] (PGCL), poly[L-lactic-co-glycolic acid] (PLGA), poly[L-lactide-co-$\epsilon$-caprolactone] (PLCL) copolymer, poly[p-dioxanone-co-$\epsilon$-caprolactone] (PDCL) copolymer, and poly[L-lactic acid-co-p-dioxanone] (PLDA) copolymer, and a raw material of hyaluronic acid in the powder form, mixing the absorbent polymer and the hyaluronic acid, spinning the absorbent polymer and the hyaluronic acid, and stretching the spun suture, wherein the hyaluronic acid is included in the absorbable suture in an amount range of more than 0.1% by weight to less than 5.0% by weight based on the total weight.

Further, the hyaluronic acid may have an average particle size range of 1 $\mu$m to 400 $\mu$m.

Further, the hyaluronic acid may have an average particle size range of 1 $\mu$m to 300 $\mu$m, and the hyaluronic acid may be contained in the absorbable suture in an amount range of 0.5% by weight or more to 4.0% by weight or less based on the total weight.

Further, the hyaluronic acid may have an average particle size range of 1 $\mu$m to 300 $\mu$m, and the hyaluronic acid may be contained in the absorbable suture in an amount range of 0.5% by weight or more to 3.0% by weight or less based on the total weight.

Further, the absorbent polymer may be composed of poly[p-dioxanone] (PDO), and poly[p-dioxanone] (PDO) may have a melting index (MI) range of 2 g/10 min or more to 16 g/10 min or less.

Further, the method may further include, before the step of performing mixing, steps of injecting the absorbent polymer raw material into a spinning chamber in a pressure range of 80 to 120 bar and a temperature range of 150°C to 190°C, and melting the injected absorbent polymer while rotating a screw inside the spinning chamber, the step of performing mixing may be characterized by injecting and mixing the hyaluronic acid when the absorbent polymer is completely melted, and the step of performing spinning may be characterized by performing spinning in a state in which the nozzle unit connected to the spinning chamber is adjusted to a temperature range of 160°C to 200°C and a pressure range of 20 bar to 60 bar.

Further, the step of performing stretching may include a first stretching step of performing stretching at a stretching ratio of 1:3 to 1:7, and a second stretching step of performing stretching at a stretching ratio of 1:1.1 to 1:2, and the method may further include a step of performing shrinking at a ratio of 1:0.5 to 1:0.9 after the step of performing stretching.

According to the absorbable suture according to the present disclosure and the method for manufacturing the same, the absorbable suture can perform a function of imparting a moisturizing effect to the skin in addition to being decomposed in the body after the procedure.

In addition, hyaluronic acid may be released continuously and in an appropriate amount even after a long time has elapsed after performing a suture procedure by including hyaluronic acid not only on the surface of the absorbable suture but also inside the absorbable suture.

In addition, it is possible to prevent deterioration in knot strength, elongation, etc., which are physical properties required of an absorbable suture while including hyaluronic acid in the absorbable suture.

## DESCRIPTION OF EXEMPLARY EMBODIMENTS

Hereinafter, preferred embodiments of the present disclosure will be described. However, the embodiments of the present disclosure can be modified in many different forms, and the scope of the present disclosure is not limited to the embodiments described below. In addition, the embodiments of the present disclosure are provided to more completely explain the present disclosure to those skilled in the art.

An absorbable suture according to one embodiment of the present disclosure includes an absorbent polymer including at least one material selected from the group consisting of poly[L-lactic acid] (PLLA), poly[lactic acid] (PLA), poly[p-dioxanone] (PDO), poly[ε-caprolactone] (PCL), poly[glycolic acid] (PGA), poly[glycolic acid- co-ε-caprolactone] (PGCL), poly[L-lactic-co-glycolic acid] (PLGA), poly[L-lactide-co-ε-caprolactone] (PLCL) copolymer, poly[p-dioxanone-co-ε-caprolactone] (PDCL) copolymer, and poly[L-lactic acid-co-p-dioxanone] (PLDA) copolymer, and hyaluronic acid contained in the absorbent polymer, wherein the hyaluronic acid is included in the absorbable suture in an amount range of more than 0.01% by weight to less than 5.0% by weight based on the total weight.

The absorbable suture of the present disclosure can be implanted into the body to maintain physical tension for a certain period of time and maintain a sutured state at the suture site. Alternatively, it can be used to pull the skin and remove wrinkles. The absorbable suture self-degrades in the body during a decomposition period of about 3 to 24 months without limitation after surgery or procedure so that there is no need for a separate medical action to remove the suture, and the decomposition period of the absorbable suture can be controlled by changing the type or content of the absorbent polymer.

Hyaluronic acid is a biosynthetic natural substance that is abundantly present in the skin of animals, etc., is a hydrophilic substance by having a lot of hydroxyl groups (-OH), and plays a role of performing moisturizing action in the skin of people, animals, or the like. It is also present in human skin, and is known to be particularly abundant in earthworm skin, and in addition to a moisturizing action, it can react with CD44 protein expressed in various epithelial cells to regulate various physiological actions.

The absorbent polymer of the present disclosure may include at least one material selected from the group consisting of poly[L-lactic acid] (PLLA), poly[lactic acid] (PLA), poly[p-dioxanone] (PDO), poly[ε-caprolactone] (PCL), poly[glycolic acid] (PGA), poly[glycolic acid- co-ε-caprolactone] (PGCL), poly[L-lactic-co-glycolic acid] (PLGA), poly[L-lactide-co-ε-caprolactone] (PLCL) copolymer, poly[p-dioxanone-co-ε-caprolactone] (PDCL) copolymer, and poly[L-lactic acid-co-p-dioxanone] (PLDA) copolymer. Further, it may include other biodegradable resins, mixtures thereof, or block copolymers without limitation in addition to the absorbent polymer described above.

In addition, the absorbable suture of the present disclosure may be composed of a monofilament, and the hyaluronic acid may be distributed inside and on the surface of the monofilament. The absorbable suture is composed of a monofilament so that it may be possible to prevent contamination due to bacterial multiplication. More specifically, when the absorbable suture is composed of a multifilament, bacteria may penetrate between several strands, increasing the probability of infection. In the case of the present disclosure, since it is composed of a monofilament, an occurrence in which bacteria penetrate between the sutures and cause infection can be minimized. In addition, it may be possible to continuously release hyaluronic acid into the skin over time even though hyaluronic acid is released immediately after the suture sutures the skin by evenly including hyaluronic acid not only on the surface of the monofilament but also inside the monofilament.

That is, the absorbable suture of the present disclosure can continuously release hyaluronic acid until decomposition is completed after the skin is sutured by evenly distributing hyaluronic acid inside and outside the suture. There may be a problem in that all of the hyaluronic acid is released in a short time after skin tissue is sutured if hyaluronic acid is simply distributed (coated) only on the surface of the suture, and there may be a problem in that hyaluronic acid is released only after a long time has passed since the suture was implanted into the skin if only the core of the suture contains hyaluronic acid separately. In the case of the present disclosure, hyaluronic acid is distributed inside and outside the suture so that it can be consistently released into the skin in the process of decomposition of the absorbable suture even if hyaluronic acid is released right after the absorbable suture is implanted so that it can consistently impart the skin moisturizing effect.

Meanwhile, the absorbable suture is characterized in that it includes the hyaluronic acid in a range of more than 0.1% by weight to less than 5.0% by weight of the total weight, for example, a range of more than 0.1% by weight to 4.0% by weight or less, a range of more than 0.1% by weight to 3.0% by weight or less, a range of 0.5% by weight or more to less than 5.0% by weight, a range of 0.5% by weight or more to 4.0% by weight or less, a range of 0.5% by weight or more to 3.0% by weight or less, a range of 1.0% by weight or more to less than 5.0% by weight, a range of 1.0% by weight or more to 4.0% by weight or less, or a range of 1.0% by weight or more to 3.0% by weight or less. Within the above range, it may be possible to minimize the decrease in knot strength and elongation required as a suture, and to achieve significant release of hyaluronic acid.

Meanwhile, when the diameter of the absorbable suture is in a range of 0.4 mm or more to 0.5 mm or less, the knot strength of the absorbable suture is in a range of 40 N or more to 60 N or less, and the knot elongation thereof is 20% or more to 40% or less. The knot strength is a force that may be supported in a tied state when the suture is tied after a procedure or surgery, and may be easily maintained when a knot is made after skin suture within the above range. For example, European standards for an absorbable suture stipulate that absorbable suture should have a knot strength of 39 N or more, and if the knot strength falls to less than 40 N, it may be difficult to use the absorbable suture as a suture. The knot strength may be, for example, in a range of 40 N or more to 60 N or less when the diameter of the absorbable suture is in a range of 0.4 mm or more to 0.5 mm or less. Meanwhile, the diameter of the absorbable suture may be in a range of 0.1 mm or more to 0.2 mm or less, a range of 0.2 mm or more to 0.3 mm or less, a range of 0.3 mm or more to 0.4 mm or less, or a range of 0.4 mm or more to 0.5 mm or less, and it may be adjusted according to the diameter desired by the user.

In addition, knot elongation refers to a ratio (%) of the length extended to the maximum load to the initial length, and when the knot elongation is 0%, it may be substantially difficult to use it as a suture. In addition, when the elongation range is satisfied, the absorbable suture maintains an appropriate tension so that it may be convenient to use it in surgeries or procedures such as skin suture.

In addition, when the diameter of the absorbable suture is in a range of 0.4 mm or more to 0.5 mm or less, the absorbable suture may have a breaking strength retention (BSR) range of 40% or more to 80% or less after 2 weeks after transplantation. Breaking strength retention (BSR) is to confirm the strength retention force in the body through an in-vitro test, and to confirm the retention strength compared to the initial strength. In the case of an absorbable suture, the strength gradually decreases as the wound heals so that the change in strength with the passage of time should be measured. Generally, the period required to heal a wound is about 2 weeks, and the strength of the suture should be maintained within this period. In order to be used as an absorbable suture, the breaking strength retention (BSR) after 2 weeks after transplantation should be 30% or more, and preferably 40% or more. If the breaking strength retention (BSR) after 2 weeks after transplantation is less than 30%, the strength of the suture decreases before the wound heals so that it may be difficult to perform its function as a suture, and thus it should be 40% or more.

Meanwhile, the absorbent polymer may be composed of poly[p-dioxanone] (PDO), and poly[p-dioxanone] (PDO) may have a melting index (MI) range of 2 g/10 min or more and 16 g/10 min or less. In the above-described melting index (MI) range, it may be possible to prevent a rapid decrease in breaking strength retention (BSR) after 2 weeks after transplantation and to enable it to be used as an absorbable suture.

Meanwhile, the present disclosure provides a method for manufacturing the above-described absorbable suture. Hereinafter, the method for manufacturing the absorbable suture will be described, and descriptions of contents overlapping with those described in the above-described absorbable suture will be omitted.

A method for manufacturing an absorbable suture according to one embodiment of the present disclosure includes the steps of preparing an absorbent polymer raw material including at least one material selected from the group consisting of poly[L-lactic acid] (PLLA), poly[lactic acid] (PLA), poly[p-dioxanone] (PDO), poly[ε-caprolactone] (PCL), poly[glycolic acid] (PGA), poly[glycolic acid- co-ε-caprolactone] (PGCL), poly[L-lactic-co-glycolic acid] (PLGA), poly[L-lactide-co-ε-caprolactone] (PLCL) copolymer, poly[p-dioxanone-co-ε-caprolactone] (PDCL) copolymer, and poly[L-lactic acid-co-p-dioxanone] (PLDA) copolymer, and a raw material of hyaluronic acid in the powder form, mixing the absorbent polymer and the hyaluronic acid, spinning the absorbent polymer and the hyaluronic acid, and stretching the spun suture, wherein the hyaluronic acid is included in the absorbable suture in an amount range of more than 0.1% by weight to less than 5.0% by weight based on the total weight.

On the other hand, while the hyaluronic acid may be included in a range of more than 0.1% by weight to less than 5.0% by weight, and it may be included, for example, in a range of more than 0.1% by weight to 4.0% by weight or less, a range of more than 0.1% by weight to 3.0% by weight or less, a range of 0.5% by weight or more to less than 5.0% by weight, a range of 0.5% by weight or more to 4.0% by weight or less, a range of 0.5% by weight or more to 3.0% by weight or less, a range of 1.0% by weight or more to less than 5.0% by weight, a range of 1.0% by weight or more to 4.0% by weight or less, or a range of 1.0% by weight or more to 3.0% by weight or less. Within the above range, it may be possible to minimize the decrease in knot strength and elongation required as a suture, and to achieve significant release of hyaluronic acid.

In addition, the hyaluronic acid may have an average particle size range of 1 μm to 400 μm, and for example, the hyaluronic acid may have an average particle size range of 1 μm to 300 μm. It may be possible to minimize the decrease in knot strength and knot elongation within the above-described particle size range. Preferably, the hyaluronic acid may have an average particle size range of 1 μm to 300 μm, and the hyaluronic acid may be included in a range of 0.5% by weight or more to 3.0% by weight or less based on the total weight of the absorbable suture.

Meanwhile, the absorbent polymer may be composed of poly[p-dioxanone] (PDO), and poly[p-dioxanone] (PDO) may have a melting index (MI) range of 2 g/10 min or more to 16 g/10 min or less. In the above-described range, it may be possible to prevent a rapid decrease in breaking strength retention (BSR) 2 weeks after implantation and to enable it to be used as an absorbable suture.

Further, before the step of performing mixing, steps of injecting the absorbent polymer raw material into a spinning chamber in a pressure range of 80 to 120 bar and a temperature range of 150°C to 190°C, and melting the injected absorbent polymer while rotating a screw inside the spinning chamber are further included. The step of performing mixing may be characterized by injecting and mixing the hyaluronic acid when the absorbent polymer is completely melted, and the step of performing spinning may be characterized by performing spinning in a state in which the nozzle unit connected to the spinning chamber is adjusted to a temperature range of 160°C to 200°C and a pressure range of 20 bar to 60 bar.

The absorbent polymer raw material may be melted while rotating the screw under conditions of a pressure range of 80 to 120 bar and a temperature range of 150°C to 190°C in the spinning chamber, and after melting is completed, hyaluronic acid is injected so that they may be more evenly mixed well with each other in the mixed raw material.

Thereafter, an absorbable suture may be manufactured by performing spinning in a temperature range of 160°C to 200°C and a pressure range of 20 bar to 60 bar through the nozzle unit connected to the spinning chamber. In addition, the step of performing stretching may include a first stretching step at a stretching ratio of 1:3 to 1:7, and a second stretching step at a stretching ratio of 1: 1.1 to 1:2.

Further, it may be characterized in that a step of performing shrinking at a ratio of 1:0.5 to 1:0.9 after the step of performing stretching is further included. The step of performing shrinking may be a process of adjusting the suture to shrink at a predetermined rate while preventing excessive shrinking from being occurred in the process of cooling the suture after the step of performing stretching.

Hereinafter, the absorbable suture of the present disclosure will be described in more detail through specific experimental data.

Experimental Example 1

Spinning of an absorbable suture was conducted by using poly[p-dioxanone] (PDO) as an absorbent polymer and mixing hyaluronic acid with it. In the spinning, hyaluronic acid was injected in a state in which poly[p-dioxanone] was all melted to further perform mixing and melting, and then spinning was performed, and absorbable sutures having an average diameter of 0.47 mm were manufactured while varying the content of hyaluronic acid. Afterwards, knot strength and knot elongation compared to the content of each hyaluronic acid were measured, and the results are shown in Table 2 below. Knot strength and knot elongation were measured using a tensile strength measuring instrument (Cometech Model QC-508). The specific measurement method was to check the regulator and air pressure (about 5 kg/cm$^2$) before testing using a tensile strength measuring instrument, and set the experimental conditions as shown in Table 1 below.

[Table 1]

| Test item | Number of tests | Limits Load (kgf) | Limits Extension (mm) | Speed (mm/mm) | Length (mm) |
|---|---|---|---|---|---|
| Knot strength | 5 times | 450 | 300 | 300 | 200 |

Knot strength and knot elongation experiments were conducted after making a knot with an absorbable suture, and knot elongation was measured simultaneously while measuring knot strength. First, the power of the tensile strength measuring instrument was turned on, and after warming up for about 20 minutes, the program was executed on the connected PC. Thereafter, after confirming the gap between the grips as 200mm, when the gap is different, the gap was adjusted using Jog, and then the air valve was opened to adjust the air pressure to about 5kg/cm2. Thereafter, the zero point of the instrument was set, the sample was fixed to the upper and lower grips, and the knot strength and knot elongation of the sample were measured by pressing the start button. The experiment was repeated five times or more, and the measured average value was recorded and measured.

[Table 2]

| Hyaluronic acid content (% by weight) | Knot strength (N) | Knot elongation (%) |
|---|---|---|
| 0.1 | 57.0 | 34.6 |
| 0.5 | 56.5 | 35,0 |
| 1.0 | 57.4 | 35.9 |
| 2.0 | 58.5 | 36.3 |
| 3.0 | 56.7 | 34.7 |
| 4.0 | 42.3 | 22.1 |
| 5.0 | 36.3 | 19.9 |
| 10.0 | 18.2 | 10.1 |
| 20.0 | 8.9 | 6.5 |

As shown in Table 2, it can be confirmed that the knot strength is maintained at 40 N or more at up to 4.0% by weight of a content of hyaluronic acid, and it can be confirmed that a decrease occurs to less than 40 N at 5.0% by weight. In the case of a general absorbable suture, the hyaluronic acid content is preferably less than 5% by weight, as the standard in Europe requires 39 N or more. Further, more preferably, the content of hyaluronic acid is desirably 4% by weight or less, and the content of hyaluronic acid is more desirably 3% by weight or less. It can be confirmed that it may be possible to prevent a rapid decrease in knot strength and knot elongation within the above-described range.

Experimental Example 2

Unlike Experimental Example 2, absorbable sutures having an average diameter of 0.47 mm were manufactured by conducting spinning while varying the average particle size of hyaluronic acid particles injected in a state in which the hyaluronic acid content was fixed such that 2% by weight of hyaluronic acid is contained in the total absorbable suture. Thereafter, the knot strength and knot elongation of the absorbable sutures were measured in the same manner as in Experimental Example 1 above, and the results are shown as Table 3.

[Table 3]

| Item | Average particle size | | | | | |
|---|---|---|---|---|---|---|
| | 50 μm | 100 μm | 200 μm | 300 μm | 400 μm | 500 μm |
| Knot strength (N) | 58.1 | 58.5 | 57.1 | 57.3 | 41.0 | 30.6 |
| Knot elongation (%) | 36.2 | 36.3 | 34.9 | 34.7 | 25.9 | 16.9 |

As shown in Table 3, when the average particle size of hyaluronic acid exceeds 400 μm, the knot strength drops to 40 N or less, and thus it may be difficult to use it as a suture. Therefore, hyaluronic acid preferably has an average particle size of 400 μm or less. In addition, it can be confirmed that when the average particle size of hyaluronic acid exceeds 300 μm, knot strength and elongation are rapidly reduced. Therefore, more preferably, the average particle size of hyaluronic acid is desirably 300 μm or less.

Experimental Example 3

Spinning of an absorbable suture was conducted by using poly[p-dioxanone] (PDO) as an absorbent polymer and mixing hyaluronic acid with it. Absorbable sutures having an average diameter of 0.47 mm were manufactured while varying the content of hyaluronic acid and the average particle size of hyaluronic acid together compared to the total weight of the absorbable suture, the knot strengths of the absorbable sutures were measured in the same manner as in Experimental Example 1 above, and the results are shown as Table 4.

[Table 4]

| Knot strength (N) | | Content (% by weight) | | | | | | | | |
|---|---|---|---|---|---|---|---|---|---|---|
| | | 0.1 | 0.5 | 1.0 | 2.0 | 3.0 | 4.0 | 5.0 | 10.0 | 20.0 |
| Average particle size (μm) | 50 | 57.3 | 55.5 | 58.5 | 58.1 | 57.0 | 42.9 | 35.1 | 20.0 | 10.5 |
| | 100 | 55.7 | 53.5 | 57.6 | 58.5 | 58.5 | 44.6 | 35.7 | 23.2 | 12.4 |
| | 200 | 54.7 | 60.0 | 56.8 | 57.1 | 57.3 | 42.1 | 36.0 | 19.1 | 11.4 |
| | 300 | 57.6 | 57.5 | 57.2 | 57.3 | 53.5 | 42.3 | 35.5 | 17.0 | 9.3 |
| | 400 | 40.1 | 40.7 | 40.2 | 41.0 | 40.7 | 30.9 | 21.5 | 13.2 | 7.4 |
| | 500 | 30.2 | 30.3 | 30.5 | 30.6 | 30.7 | 20.7 | 18.9 | 10.2 | 6.3 |

As shown in Table 4, when the content of hyaluronic acid is in a range of less than 5.0% by weight, the knot strength is prevented from falling to 40 N or less so that the absorbable suture can be used as a suture, and it can be confirmed that a range of 4.0% by weight or less is preferable. More preferably, when the content of hyaluronic acid is 3.0% by weight or less, it can be confirmed that a rapid decrease in knot strength can be prevented. In addition, it can be confirmed that the knot strength can be stably secured in a range of the average particle size of hyaluronic acid of 400 μm or less, and it can be confirmed that a range of the average particle size of hyaluronic acid of 300 μm or less is preferable by preventing a rapid decrease in knot strength. Meanwhile, when the average particle size of hyaluronic acid is in a range of 400 μm or less and the content of hyaluronic acid is less than 5.0% by weight, preferably 4.0% by weight or less, or more preferably 3.0% by weight or less, the absorbable suture can perform its function as a suture while minimizing the decrease in knot strength. In addition, when the average particle size of hyaluronic acid is in a range of 300 μm or less and the content of hyaluronic acid is less than 5.0% by weight, preferably 4.0% by weight or less, or more preferably 3.0% by weight or less, the absorbable suture can perform its function as a suture while minimizing the decrease in knot strength.

Experimental Example 4

Spinning of an absorbable suture was conducted by using poly[p-dioxanone] (PDO) as an absorbent polymer and mixing hyaluronic acid with it. Absorbable sutures having an average diameter of 0.47 mm were manufactured while varying the content of hyaluronic acid compared to the total weight of the absorbable suture, the detection amounts of hyaluronic acid released from the respective manufactured absorbable sutures during a period over 8 weeks were measured, and the detection amounts measured after 1 week, 2 weeks, 3 weeks, 4 weeks, 5 weeks, 6 weeks, 7 weeks, and 8 weeks are shown in Table 5 according to the respective contents. The detection amounts of hyaluronic acid were measured using a UV Spectrometer (Jasco V-700 model), and a wavelength of 260 nm that can measure the concentration of DNA was used as a wavelength used. An absorbable suture using poly[p-dioxanone] (PDO) to which hyaluronic acid was not added was used as a control group used in the test.

The test was proceeded by turning on a UV spectrometer, adjusting the zero point using Autozero, and then preparing a diluted solution (PBS buffer), and measuring the absorbance with the UV Spectrometer by using the diluted solution as a blank. Thereafter, control group samples (poly[p-dioxanone] sutures in which hyaluronic acid is not contained) dissolved in the diluted solution were contained in a quartz cell and measured with the UV spectrometer, samples mixed with absorbable sutures manufactured for the respective hyaluronic acid contents were dissolved in the diluted solution, contained in a quartz cell, and respectively measured with the UV spectrometer, and the values obtained by subtracting the absorbance value of the control group from the absorbance values of the samples were used as the resultant values. Meanwhile, as a standard solution, a solution in which only hyaluronic acid was dissolved was measured with the UV spectrometer to confirm the absorbance value for each concentration, and concentration values were calculated by comparing the resultant values of the samples with reference to the absorbance value of the standard solution.

[Table 5]

| Content (% by weight) | 0.01 | 0.05 | 0.1 | 0.5 | 1.0 | 3.0 | 5.0 | 10.0 |
|---|---|---|---|---|---|---|---|---|
| Time (in weeks) | Release amount | | | | | | | |
| 1 week | 2.6 | 8.6 | 12.3 | 126.6 | 249.5 | 750.5 | 1244.4 | 2400.5 |
| 2 weeks | 4.3 | 14.3 | 20.4 | 210.1 | 413.9 | 1247.1 | 2069.5 | 4141.7 |
| 3 weeks | 4.1 | 13.8 | 19.7 | 202.9 | 399.7 | 1200.1 | 1999.0 | 3993.0 |
| 4 weeks | 3.2 | 10.6 | 15.1 | 155.5 | 306.3 | 919.0 | 1535.1 | 3070.0 |
| 5 weeks | 4.2 | 13.9 | 19.9 | 204.9 | 403.7 | 1211.0 | 2334.5 | 4110.0 |
| 6 weeks | 4.8 | 16.2 | 23.1 | 237.9 | 468.7 | 1406.3 | 2018.8 | 4688.3 |
| 7 weeks | 6.4 | 21.3 | 30.4 | 313.1 | 616.8 | 1854.5 | 3085.5 | 6008.5 |
| 8 weeks | 8.7 | 29.2 | 41.7 | 429.5 | 846.1 | 2580.0 | 4333.0 | 8500.1 |

As shown in Table 5, when the content of hyaluronic acid is 0.01, 0.05, and 0.1% by weight, it can be confirmed that the hyaluronic acid is substantially released at a very insignificant level, and it can be confirmed that the release amount is rapidly increased at the content of 0.5% by weight compared to 0.1% by weight. Therefore, it is preferable that the content of hyaluronic acid is more than 0.1% by weight. More preferably in a range of 0.5% by weight or more, the amount of hyaluronic acid released may be relatively increased, and for example, in a range of 1.0% by weight or more, hyaluronic acid may be released more significantly.

Experimental Example 5

Spinning of an absorbable suture was conducted by using poly[p-dioxanone] (PDO) as an absorbent polymer and mixing hyaluronic acid with it. Absorbable sutures having an average diameter of 0.47 mm were manufactured while varying the melting index (MI) of poly[p-dioxanone], and breaking strength retention (BSR) values after 2 weeks after transplantation into the skin using the respective manufactured absorbable sutures were measured, and the results are shown in Table 6 below.

A specific measurement method for measuring breaking strength retention (BSR) after 2 weeks after transplantation into the skin was to first put into distilled water in a 1L volumetric flask, 5 BSR tablets (Sigma Aldrich phosphate buffered saline tablet) were added thereinto, and the mixture was completely dissolved using Spinbar and a magnetic stirrer to prepare a buffer solution of pH 7.4. Thereafter, two 3 m absorbable suture samples to be measured were prepared, and were each placed in a 50ml graduated centrifugal tube. Thereafter, about 40 ml of the prepared buffer solution was put into the graduated centrifugal tube so that the sample contained in the graduated centrifugal tube was sufficiently submerged, and the lid of the graduated centrifugal tube was closed.

Next, distilled water was poured into and prepared in a shaking water bath, the temperature of the bath was set to 37°C $\pm$ 0.3, and the shaking speed was set to 30 rpm, and the graduated centrifugal tube containing the sample was immersed in the shaking water bath. Meanwhile, the knot strength was measured on the remaining one sample as day 0, and the result was recorded.

Meanwhile, when the corresponding immersion days (2 weeks) were reached, the sample was taken out of the graduated centrifugal tube and the knot strength (EP knot) of the sample was measured using a tensile strength measuring instrument (Cometech Model QC-508). The measurement conditions of the tensile strength measuring instrument were a sample length of 30 cm or more, a grip interval of 200 mm, and a speed of 300 mm/min, the measurement results were recorded, and the knot strength retention rate was calculated based on the knot strength at day 0. The strength retention rate (%) is a value obtained by dividing the knot strength (kgf) after passage of a specific time by the initial knot strength (kgf) and multiplying by 100, and the specific expression is as follows.

$$\text{Strength retention rate (\%)} = \frac{\text{Knot strength (kgf) after passage of a specific time}}{\text{Initial knot strength (kgf)}} \ 100$$

[Table 6]

| MI(g/10min) | BSR (%) after 2 weeks |
|---|---|
| 2 | 68.0 |
| 8 | 65.0 |
| 10 | 60.0 |
| 14 | 61.0 |
| 16 | 58.0 |
| 18 | 43.0 |
| 20 | 40.0 |
| 22 | 35.0 |

Breaking strength retention (BSR) after 2 weeks after transplantation in the skin is to confirm the strength retention in the body through an in-vitro test, which is to confirm the retention strength compared to the initial strength. In the case of absorbable sutures, the strength gradually decreases as the wound heals so that the change in strength with the passage of time should be measured. Generally, the period required to heal a wound is about 2 weeks, and the strength of the suture should be maintained within this period. In order for the absorbable sutures to be used as an absorbable suture, it is preferable that the strength retention rate (BSR) should be 40% or more after 2 weeks after transplantation. Therefore, as can be confirmed in Table 6 above, a melting index (MI) of poly[p-dioxanone] (PDO) in the absorbable suture may be in a range of 1 g/10 min or more to 20 g/10 min or less, preferably in a range of 2 g/10 min or more to 18 g/10 min or less. Meanwhile, compared to the BSR numerical value when the melting index of poly[p-dioxanone] is in a range of 2 g/10 min or more to 16 g/10 min or less, the BSR numerical value decreases rapidly at a melting index of 18 g/10 min so that, most preferably, an excellent BSR index may be satisfied when the melting index of poly[p-dioxanone] satisfies a range of 2 g/10 min or more to 16 g/10 min or less.

Experimental Example 6

Spinning of an absorbable suture was conducted by using poly[p-dioxanone] (PDO) as an absorbent polymer and mixing hyaluronic acid with it. Absorbable sutures having an average diameter of 0.47 mm were manufactured while varying the melting index (MI) of poly[p-dioxanone] (PDO) and the content of hyaluronic acid, and breaking strength retention (BSR) values 2 weeks after transplantation into the skin using the respective manufactured absorbable sutures were measured. The results of the measured BSR are shown in Table 7 below. Strength retention rate (BSR) was measured in the same manner as in Experimental Example 5.

[Table 7]

| After 2 weeks of BSR (%) | | Hyaluronic acid content (% by weight) | | | | | | | |
|---|---|---|---|---|---|---|---|---|---|
| | | 0.01 | 0.05 | 0.1 | 0.5 | 1.0 | 3.0 | 5.0 | 10.0 |
| MI (g/min) | 2 | 78.0% | 80.0% | 76.0% | 78.0% | 77.0% | 68.0% | 65.0% | 60.0% |
| | 8 | 75.0% | 77.0% | 73.0% | 75.0% | 75.0% | 65.0% | 66.0% | 58.0% |
| | 10 | 70.0% | 69.0% | 68.0% | 66.0% | 65.0% | 60.0% | 61.0% | 57.0% |
| | 14 | 69.0% | 67.0% | 65.0% | 63.0% | 65.0% | 61.0% | 60.0% | 55.0% |
| | 16 | 66.0% | 61.0% | 63.0% | 66.0% | 62.0% | 58.0% | 53.0% | 50.0% |
| | 18 | 58.0% | 60.0% | 59.0% | 49.0% | 45.0% | 43.0% | 41.0% | 39.0% |
| | 20 | 51.0% | 52.0% | 48.0% | 41.0% | 42.0% | 40.0% | 39.0% | 35.0% |
| | 22 | 43.0% | 40.0% | 41.0% | 38.0% | 37.0% | 35.0% | 31.0% | 20.0% |

Since, if the BSR after 2 weeks is less than 40%, the strength of the suture decreases before the wound heals so that it may be difficult to perform its function as a suture, the minimally required numerical value is required to be 40% or more. Looking through Table 7, the melting index (MI) of poly[p-dioxanone] (PDO) may be in a range of 1 g/10 min or more to 20 g/10 min or less, and preferably a range of 2 g/10 min or more to 18 g/10 min or less. Meanwhile, compared to the BSR numerical value when the melting index of poly[p-dioxanone] is in a range of 2 g/10 min or more to 16 g/10 min or less, the BSR value decreases rapidly at a melting index of 18 g/10 min, and thus most preferably, an excellent BSR value may be satisfied when the melting index of poly[p-dioxanone] satisfies the range of 2 g/10 min or more to 16 g/10 min or less. In addition, the content of hyaluronic acid is preferably in a range of less than 5.0% by weight, more preferably a range of 4.0% by weight or less, and further more preferably a range of 3.0% by weight or less. Specifically, it is preferable that the melting index of poly[p-dioxanone] satisfies a range of 2 g/10 min or more to 16 g/10 min or less while the hyaluronic acid satisfies a range of more than 0.1% by weight to less than 5.0% by weight, a range of more than 0.1% by weight to 4.0% by weight or less, a range of more than 0.1% by weight to 3.0% by weight or less, a range of 0.5% by weight or more to less than 5.0% by weight, a range of 0.5% by weight or more to 4.0% by weight or less, a range of 0.5% by weight or more to 3.0% by weight or less, a range of 1.0% by weight or more to less than 5.0% by weight, a range of 1.0% by weight or more to 4.0% by weight or less, or a range of 1.0% by weight or more to 3.0% by weight or less.

Although the embodiments of the present disclosure have been described in detail above, the scope of rights of the present disclosure is not limited thereto, and it will be obvious to those of ordinary skill in the art that various modifications and variations are possible without departing from the technical spirit of the present disclosure described in the claims.

**Claims**

1. An absorbable suture comprising:

   an absorbent polymer including at least one material selected from the group consisting of poly[L-lactic acid] (PLLA), poly[lactic acid] (PLA), poly[p-dioxanone] (PDO), poly[ε-caprolactone] (PCL), poly[glycolic acid] (PGA), poly[glycolic acid- co-ε-caprolactone] (PGCL), poly[L-lactic-co-glycolic acid] (PLGA), poly[L-lactide-co-ε-caprolactone] (PLCL) copolymer, poly[p-dioxanone-co-ε-caprolactone] (PDCL) copolymer, and poly[L-lactic acid-co-p-dioxanone] (PLDA) copolymer; and

hyaluronic acid contained in the absorbent polymer;
wherein the hyaluronic acid is contained in the absorbable suture in an amount range of more than 0.1% by weight to less than 5.0% by weight based on the total weight.

2. The absorbable suture of claim 1, wherein the absorbable suture is composed of a monofilament, and the hyaluronic acid is distributed inside and on the surface of the monofilament.

3. The absorbable suture of claim 1, wherein the hyaluronic acid is contained in a range of 0.5% by weight or more to less than 5.0% by weight of the total weight of the absorbable suture.

4. The absorbable suture of claim 1, wherein the hyaluronic acid is contained in a range of 0.5% by weight or more to 4.0% by weight or less of the total weight of the absorbable suture.

5. The absorbable suture of claim 1, wherein the hyaluronic acid is contained in a range of 0.5% by weight or more to 3.0% by weight or less of the total weight of the absorbable suture.

6. The absorbable suture of claim 1, wherein the absorbable suture has a breaking strength retention (BSR) range of 40% or more to 80% or less after 2 weeks after transplantation.

7. The absorbable suture of claim 1, wherein the absorbent polymer is composed of poly[p-dioxanone] (PDO), and poly[p-dioxanone] (PDO) has a melting index (MI) range of 2 g/10 min or more to 16 g/10 min or less.

8. A method for manufacturing an absorbable suture, the method comprising the steps of:

   preparing an absorbent polymer raw material including at least one material selected from the group consisting of poly[L-lactic acid] (PLLA), poly[lactic acid] (PLA), poly[p-dioxanone] (PDO), poly[$\varepsilon$-caprolactone] (PCL), poly[glycolic acid] (PGA), poly[glycolic acid- co-$\varepsilon$-caprolactone (PGCL), poly[L-lactic-co-glycolic acid] (PLGA), poly[L-lactide-co-$\varepsilon$-caprolactone] (PLCL) copolymer, poly[p-dioxanone-co-$\varepsilon$-caprolactone] (PDCL) copolymer, and poly[L-lactic acid-co-p-dioxanone] (PLDA) copolymer, and a raw material of hyaluronic acid in the powder form;
   mixing the absorbent polymer and the hyaluronic acid;
   spinning the absorbent polymer and the hyaluronic acid; and
   stretching the spun suture;
   wherein the hyaluronic acid is contained in the absorbable suture in an amount range of more than 0.1% by weight to less than 5.0% by weight based on the total weight.

9. The method of claim 8, wherein the hyaluronic acid has an average particle size range of 1 $\mu$m to 400 $\mu$m.

10. The method of claim 8, wherein the hyaluronic acid has an average particle size range of 1 $\mu$m to 300 $\mu$m, and the hyaluronic acid is contained in the absorbable suture in an amount range of 0.5% by weight or more to 4.0% by weight or less based on the total weight.

11. The method of claim 8, wherein the hyaluronic acid has an average particle size range of 1 $\mu$m to 300 $\mu$m, and the hyaluronic acid is contained in the absorbable suture in an amount range of 0.5% by weight or more to 3.0% by weight or less based on the total weight.

12. The method of claim 8, wherein the absorbent polymer is composed of poly[p-dioxanone] (PDO), and poly[p-dioxanone] (PDO) has a melting index (MI) range of 2 g/10 min or more to 16 g/10 min or less.

13. The method of claim 8, further comprising, before the step of performing mixing, steps of:

   injecting the absorbent polymer raw material into a spinning chamber in a pressure range of 80 to 120 bar and a temperature range of 150°C to 190°C; and
   melting the injected absorbent polymer while rotating a screw inside the spinning chamber;
   wherein the step of performing mixing is **characterized by** injecting and mixing the hyaluronic acid when the absorbent polymer is completely melted, and the step of performing spinning is **characterized by** performing spinning in a state in which the nozzle unit connected to the spinning chamber is adjusted to a temperature range of 160°C to 200°C and a pressure range of 20 bar to 60 bar.

14. The method of claim 13, wherein the step of performing stretching includes a first stretching step of performing stretching at a stretching ratio of 1:3 to 1:7, a second stretching step of performing stretching at a stretching ratio of 1:1.1 to 1:2, and the method further includes a step of performing shrinking at a ratio of 1:0.5 to 1:0.9 after the step of performing stretching.

## INTERNATIONAL SEARCH REPORT

| International application No. |
|---|
| **PCT/KR2022/003958** |

**A. CLASSIFICATION OF SUBJECT MATTER**

**A61L 17/10**(2006.01)i; **A61L 17/12**(2006.01)i; **A61L 17/00**(2006.01)i; **A61B 17/06**(2006.01)i; **A61B 17/00**(2006.01)i

According to International Patent Classification (IPC) or to both national classification and IPC

**B. FIELDS SEARCHED**

Minimum documentation searched (classification system followed by classification symbols)

A61L 17/10(2006.01); A61B 17/00(2006.01); A61B 17/06(2006.01); A61F 2/00(2006.01); A61L 17/14(2006.01); A61L 27/14(2006.01); A61L 27/54(2006.01); B29C 55/00(2006.01)

Documentation searched other than minimum documentation to the extent that such documents are included in the fields searched

Korean utility models and applications for utility models: IPC as above
Japanese utility models and applications for utility models: IPC as above

Electronic data base consulted during the international search (name of data base and, where practicable, search terms used)

eKOMPASS (KIPO internal) & keywords: 히알루론산(hyaluronic acid), 봉합사(suture), 흡수성 고분자(absorbable polymer)

**C. DOCUMENTS CONSIDERED TO BE RELEVANT**

| Category* | Citation of document, with indication, where appropriate, of the relevant passages | Relevant to claim No. |
|---|---|---|
| X | KR 10-2018-0109914 A (AMIR, Avraham) 08 October 2018 (2018-10-08)<br>See claims 1, 6, 7 and 23; and paragraphs [0022]-[0061]. | 1-7 |
| Y | | 8-14 |
| Y | US 2016-0166727 A1 (TEPHA, INC.) 16 June 2016 (2016-06-16)<br>See paragraphs [0086]-[0097]. | 8-14 |
| A | KR 10-2016-0107781 A (DEXLEVO INC. et al.) 19 September 2016 (2016-09-19)<br>See entire document. | 1-14 |
| A | KR 10-2020-0033487 A (G-MEDIENCE CO., LTD.) 30 March 2020 (2020-03-30)<br>See entire document. | 1-14 |
| A | KR 10-2020-0007698 A (SAMYANG BIOPHARMACEUTICALS CORPORATION) 22 January 2020 (2020-01-22)<br>See entire document. | 1-14 |

☐ Further documents are listed in the continuation of Box C.　　☑ See patent family annex.

| | |
|---|---|
| * Special categories of cited documents:<br>"A" document defining the general state of the art which is not considered to be of particular relevance<br>"D" document cited by the applicant in the international application<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search | Date of mailing of the international search report |
|---|---|
| **17 October 2022** | **17 October 2022** |

| Name and mailing address of the ISA/KR | Authorized officer |
|---|---|
| **Korean Intellectual Property Office**<br>**Government Complex-Daejeon Building 4, 189 Cheongsa-ro, Seo-gu, Daejeon 35208** | |
| Facsimile No. **+82-42-481-8578** | Telephone No. |

Form PCT/ISA/210 (second sheet) (July 2019)

**INTERNATIONAL SEARCH REPORT**
Information on patent family members

International application No.

**PCT/KR2022/003958**

| Patent document cited in search report | | | Publication date (day/month/year) | Patent family member(s) | | | Publication date (day/month/year) |
|---|---|---|---|---|---|---|---|
| KR | 10-2018-0109914 | A | 08 October 2018 | EP | 3400028 | A1 | 14 November 2018 |
| | | | | EP | 3400028 | A4 | 25 September 2019 |
| | | | | JP | 2019-503832 | A | 14 February 2019 |
| | | | | US | 2019-0150922 | A1 | 23 May 2019 |
| | | | | WO | 2017-118972 | A1 | 13 July 2017 |
| US | 2016-0166727 | A1 | 16 June 2016 | EP | 3230500 | A1 | 18 October 2017 |
| | | | | US | 10227713 | B2 | 12 March 2019 |
| | | | | US | 10590566 | B2 | 17 March 2020 |
| | | | | US | 2017-0081784 | A1 | 23 March 2017 |
| | | | | US | 2018-0209073 | A1 | 26 July 2018 |
| | | | | US | 2020-0240044 | A1 | 30 July 2020 |
| | | | | US | 9555155 | B2 | 31 January 2017 |
| | | | | WO | 2016-094669 | A1 | 16 June 2016 |
| KR | 10-2016-0107781 | A | 19 September 2016 | KR | 10-1696646 | B1 | 17 January 2017 |
| KR | 10-2020-0033487 | A | 30 March 2020 | None | | | |
| KR | 10-2020-0007698 | A | 22 January 2020 | KR | 10-2378824 | B1 | 25 March 2022 |

Form PCT/ISA/210 (patent family annex) (July 2019)